# EUROPEAN PATENT APPLICATION

(11) **EP 3 417 904 A1**
(43) Date of publication of application: **26.12.2018**
(21) Application number: 18275087.7
(22) Date of filing: 21.06.2018
(51) Int. Cl.: A61M 25/10, A61F 2/954, A61F 2/958

(54) **DUAL BALLOON FOR LUMEN SUPPORT OR DILATION**

(30) Priority: 23.06.2017 US 201715631772
(71) Applicant: Cook Medical Technologies LLC, Bloomington, IN 47404 (US)
(72) Inventor: Eaton, Elizabeth A., Bloomington, IN 47401 (US)
(74) Representative: Williams Powell

(57) **Abstract**

A system for providing lumen support and dilation includes a balloon catheter having an inner balloon (119) and an outer balloon (118) bonded to a shaft (116), with the outer balloon subsuming the inner balloon and defining an intermediate space therebetween. The outer balloon has holes formed in an outer wall, with the inner balloon being free from holes. The shaft includes a delivery lumen in communication with the intermediate space. Daughter balloons (150) are deliverable into the intermediate space through the delivery lumen and into engagement with holes formed in the outer balloon, where the daughter balloons are inflated and retained by the holes of the outer balloon. The catheter may include one or more preloaded wires (140) extending through the delivery lumen and into the intermediate space and through the holes, such that the daughter balloons are deliverable over the wires into the corresponding hole.

## Description

### TECHNICAL FIELD

This invention relates to endoluminal medical devices for introduction into the human or animal body for treatment of endovascular disease.

### BACKGROUND OF THE INVENTION

The functional vessels of human and animal bodies, such as blood vessels and ducts, occasionally weaken or even rupture. For example, the aortic wall can weaken, resulting in an aneurysm, or it may develop a tear in one of the layers of the aortic wall resulting in an aortic dissection.

One common surgical intervention for weakened, aneurysmal or ruptured passageways or ducts involves the introduction of a compliant balloon into the damaged blood vessel.

One type of surgical intervention utilizing the insertion of a balloon into the patient's vasculature is Percutaneous Transluminal Angioplasty (PTA), often referred to simply as angioplasty, for opening up a blocked blood vessel. This procedure involves the insertion of a balloon catheter through the vasculature and to the desired location of the blockage. The balloon is inflated and deflated at the location of the blockage, thereby opening up the blood vessel.

Another type of surgical intervention involving balloons is a procedure where a balloon catheter is introduced toward a blood vessel, such as the aorta, to repair a dissection that has occurred. In this procedure, a compliant balloon is introduced to a location adjacent the tear in the vessel wall, and the balloon is inflated to block blood flow through the "true" lumen of the blood vessel, allowing the filling/thrombosis of the "false" lumen.

In many cases, however, the damaged or defected portion of the vasculature may include a branch vessel branching from the main vessel or may be near one of these branch vessels. For example, in the case of the abdominal aorta, there are at least three major branch vessels, including the celiac, mesenteric, and renal arteries, as well as other vessels, leading to various other body organs.

Thus, in the case of a vessel blockage, it can be difficult to open up the blockage near the branch vessel or in the branch vessel itself with a traditional balloon, and it may be undesirable to inflate a balloon across an opening of a branch vessel to repair a blockage in a main vessel. In the case of a vessel dissection, inflating the balloon across a branch vessel opening may not effectively block the true lumen.

### SUMMARY OF THE INVENTION

The present invention seeks to provide an improved medical system. There is also taught a method of supporting or dilating a body vessel.

According to an aspect of the present invention, there is provided a medical system having a balloon catheter including a shaft and an inner balloon bonded to the shaft and an outer balloon bonded to the shaft and subsuming the first balloon. A first inflation lumen extends through the shaft and is in fluid communication with an interior cavity of the inner balloon. An intermediate space is defined between an inner surface of the outer balloon and an outer surface of the inner balloon.

The shaft includes at least one delivery lumen extending longitudinally through the shaft that is in fluid communication with the intermediate space. The delivery lumen is configured to deliver medical devices through the shaft and into the intermediate space. At least one hole is defined in an outer wall of the outer balloon.

The system preferably further includes at least one daughter balloon that is moveable through the delivery lumen and is configured to engage the hole of the outer balloon when the daughter balloon is inflated and expanded. The inner balloon may be a compliant balloon, and the daughter balloon may be minimally compliant.

In one approach, the shaft may include a second inflation lumen that communicates with the intermediate space. The balloons may be independently inflatable via the different inflation lumens.

The system may further include wires extending through the delivery lumen and into the intermediate space. The wires may terminate within the intermediate space, or they may extend through the holes in the outer balloon. The wires may be detachably retained on the shaft outside of the outer balloon. The holes may be pre-defined in the outer balloon, or the holes may be defined by puncturing the outer balloon with the wires.

The system may include multiple delivery lumens rather than a single delivery lumen, and may include wires extending through each of the delivery lumens. The system may likewise include multiple daughter balloons that are deliverable through the different delivery lumens over the different wires.

In one approach, inflation of the inner balloon expands the outer balloon. When the daughter balloons are expanded within the holes of the outer balloon, the expansion may seal the holes in the outer balloon. The outer balloon may include reinforcing structure in addition to a compliant material of the balloon.

In another example, a medical system is provided having a balloon catheter including a shaft and an inner balloon bonded to the shaft and an outer balloon bonded to the shaft and subsuming the first balloon. A first inflation lumen extends through the shaft and is in fluid communication with an interior cavity of the inner balloon. An intermediate space is defined between an inner surface of the outer balloon and an outer surface of the inner balloon.

The shaft includes at least one delivery lumen extending longitudinally through the shaft that is in fluid communication with the intermediate space. The delivery lumen is configured to deliver medical devices through the shaft and into the intermediate space.

The system preferably further includes at least one daughter balloon that is moveable through the delivery lumen. The system includes at least one wire extending through the delivery lumen and into the intermediate space. The daughter balloon is deliverable to the intermediate space over the wire.

The system may include a delivery configuration and a deployed configuration, where the inner balloon and outer balloon are in a compressed state I the delivery configuration. In the deployed configuration, at least one of the inner balloon or the outer balloon are inflated, and the at least one daughter balloon extends through a corresponding hole defined in the outer balloon and is supported by the outer balloon when the daughter balloon is inflated.

The hole corresponding to the daughter balloon may be pre-defined through the outer balloon in the delivery configuration. In another approach, the wire may terminate within the intermediate space in the delivery configuration, and the hole corresponding to the daughter balloon is defined in response to piercing the outer balloon with the wire.

The outer balloon may include reinforcing structure in addition to a compliant material of the balloon.

A method of supporting or dilating a body vessel includes delivering, to a body vessel, a balloon catheter having an inner balloon and an outer balloon each bonded to a shaft. The outer balloon subsumes the inner balloon and defines an intermediate space therebetween. The shaft includes an inflation lumen in communication with the inner balloon and at least one delivery lumen in communication with the intermediate space.

The method preferably further includes delivering at least one daughter balloon through the delivery lumen into the intermediate space and into engagement with a hole formed in an outer wall of the outer balloon. The daughter balloon is inflated against the hole in the outer balloon. The inner balloon is inflated against the outer balloon, which reduces the intermediate space. The outer balloon is expanded into engagement with the vessel wall.

The balloon catheter may include at least one wire extending through the delivery lumen and into the intermediate space. The method includes routing the wire to a desired location outside of the outer balloon. The wire extends through the hole from the intermediate space. The daughter balloon may include a wire lumen, and the daughter balloon is delivered over the wire and through the intermediate space into engagement with the hole through which the wire extends.

The outer balloon is inflatable via a second inflation lumen extending through the shaft, and the method may include inflating the outer balloon and increasing the intermediate space. The daughter balloons may be inflated prior to inflating the inner balloon, and the outer balloon expands in response to inflation of the inner balloon.

The foregoing paragraphs have been provided by way of general introduction, and are not intended to limit the scope of the following claims. The presently preferred embodiments, together with further advantages, will be best understood by reference to the following detailed description taken in conjunction with the accompanying drawings.

### BRIEF DESCRIPTION OF THE DRAWINGS

Embodiments of the present invention are described below, by way of example only, with reference to the accompanying drawings, in which:
Figure 1 shows a balloon catheter having an inflatable balloon having holes formed in an outer wall, and a shaft having a delivery lumen in communication with the interior of the balloon, and wires extending out of the delivery lumen and through the interior of the balloon and out of the holes;
Figure 2A is a cross-sectional view of one embodiment of the shaft, showing a delivery lumen, an inflation lumen for the balloon, and a wire lumen for delivering the balloon catheter over a guidewire;
Figure 2B is another embodiment, where the shaft includes a delivery lumen and a guidewire lumen;
Figure 2C is a schematic cross-sectional view illustrating a tube that extends from the insertion end of the shaft that is integral with the shaft, with the guidewire lumen extending through the tube;
Figure 3 is a schematic view of a daughter balloon having a shaft and an inflation lumen;
Figure 4 is a cross-sectional view of the shaft of the daughter balloon;
Figure 5 is a schematic view of the daughter balloon delivered over the wire through the delivery lumen and into the balloon and out of the hole in the balloon, and inflated into engagement with the hole;
Figure 5A illustrates a reinforcing band extending around the hole in the balloon;
Figure 5B illustrates a mesh material embedded in the balloon wall around the hole in the balloon;
Figure 6 is a view of multiple daughter balloons delivered into the holes of the balloon, and the balloon inflated after each of the daughter balloons have been delivered and inflated;
Figure 7 is a cross-sectional view of the balloon catheter and the balloon in a compressed state within a delivery sheath, with wires preloaded in a delivery state;
Figure 8 illustrates the balloon catheter delivered to a body vessel and exposed from the delivery sheath, with the wires being routed into adjacent branch vessels from the holes in the balloon;
Figure 9 illustrates the daughter balloons delivered across the holes and inflated into engagement with the holes and extending into the branch vessels;
Figure 10 illustrates the balloon in an inflated condition and expanded into contact with the body vessel wall with the daughter balloons inflated into engagement with the branch vessels;
Figure 11 illustrates another embodiment of a balloon catheter having a shaft and an inner balloon and an outer balloon bonded to the shaft, with wires extending through a delivery lumen of the shaft and into the intermediate space, with the wires extending through holes formed in the outer balloon;
Figure 12 illustrates a cross-section of the shaft illustrating two inflation lumens, one for each balloon, a wire lumen, and three delivery lumens;
Figure 13 illustrates a cross-section of another embodiment of the shaft having the lumens of Figure 12 oriented differently, and four delivery lumens;
Figure 14 is cross-sectional representation of the inner balloon and the outer balloon, with daughter balloons extending through the holes of the outer balloon after being delivering into the intermediate space from the delivery lumen;
Figure 15 illustrates a cross-sectional representation of the daughter balloon having a shaft with dual inflation lumens and a wire lumen;
Figure 16 is a schematic view of the outer balloon including a reinforcing mesh;
Figure 17 is a schematic view of the wires terminating within the intermediate space, and the outer balloon having no pre-defined holes;
Figure 18 is a schematic view of the wires extending through pre-defined holes in the outer balloon and being retained on the balloon catheter;
Figure 19 illustrates the balloon catheter in deployed state, with the wires routed into adjacent branch vessels and the daughter balloons delivered to their desired locations, with the inner and outer balloons in a deflated state; and
Figure 20 illustrates the inner balloon in an inflated state, and the inner and outer balloons expanded, with the outer balloon engaging the vessel wall in response to inflation of the inner balloon.

### DETAILED DESCRIPTION

Unless specified otherwise, all technical and scientific terms used herein have the same meaning as commonly understood to one of ordinary skill in the art to which this invention belongs.

The term "distal" means a location or direction that is, or a portion of a device that when implanted is further downstream in the direction of or with respect to blood flow. In the case of aortic intervention, distal means a location further away from the heart. In a trans-femoral approach, the distal end of a device is the end that is closer to the operator.

The term "proximal" means a location or direction that is, or a portion of a device that when implanted is further upstream in the direction of or with respect to blood flow. In the case of aortic intervention, proximal means a location closer to the heart. In a trans-femoral approach, the proximal end of a device is the insertion end of the device.

The term "fenestration" means an opening provided through a surface of a prosthesis from the interior of the prosthesis to the exterior of the prostheses and may have a variety of geometries, including circular, semi-circular, oval, oblong, as well as other geometries.

The term "biocompatible" refers to a material that is substantially non-toxic in the in vivo environment of its intended use, and that is not substantially rejected by the patient's physiological system (i.e., is non-antigenic). Examples of biocompatible materials from which textile graft material can be formed include, without limitation, polyesters, such as polyethylene terephthalate; fluorinated polymers, such as polytetrafluoroethylene (PTFE) and fibers of expanded PTFE, and polyurethanes. In addition, materials that are not inherently biocompatible may be subjected to surface modifications in order to render the materials biocompatible. Examples of surface modifications include graft polymerization of biocompatible polymers on the materials surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Fibers suitable for making textile grafts include polyethylene, polypropylene, polyaramids, polyacrylonitrile, nylon, and cellulose, in addition to the polyesters, fluorinated polymers, and polyurethanes as listed above. Furthermore, bioremodelable materials may also be used singly or in combination with the aforementioned polymer materials. The textile may be made of one or more polymers that do not require treatment or modification to be biocompatible. The graft may be constructed from woven multifilament polyester, for example and without limitation, Dacron™, produced by DuPONT. Dacron™ is known to be sufficiently biologically inert, non-biodegradable, and durable to permit safe insertion inside the human body.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "tubular" refers to the general shape of an endoluminal device which allows the module to carry fluid along a distance or fit within a tubular structure such as an artery. Tubular prosthetic devices include single, branched, and bifurcated devices. Tubular may refer to any shape including, but not limited to, tapered, cylindrical, curvilinear, or any combination thereof. A tubular device may have a cross-sectional shape that is, circular, substantially circular or the like. However, it should be understood that the cross-sectional shape is not limited thereto, and other shapes, such as, for example, hexagonal, pentagonal, octagonal, or the like are contemplated. The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "branch vessel" refers to a vessel that branches off from a main vessel. Examples are the celiac and renal arteries which are branch vessels to the aorta (i.e., the main vessel in this context). As another example, the hypogastric artery is a branch vessel to the common iliac, which is a main vessel in this context. Thus, it should be seen that "branch vessel" and "main vessel" are relative terms.

"Longitudinally" refers to a direction, position or length substantially parallel with a longitudinal axis of a reference, and is the length-wise component of the helical orientation.

"Circumferentially" refers to a direction, position, or length that encircles a longitudinal axis of reference. The term "circumferential" is not restricted to a full 360° circumferential turn or to a constant radius.

The terms "patient," "subject," and "recipient" as used in this application refer to any animal, especially humans.

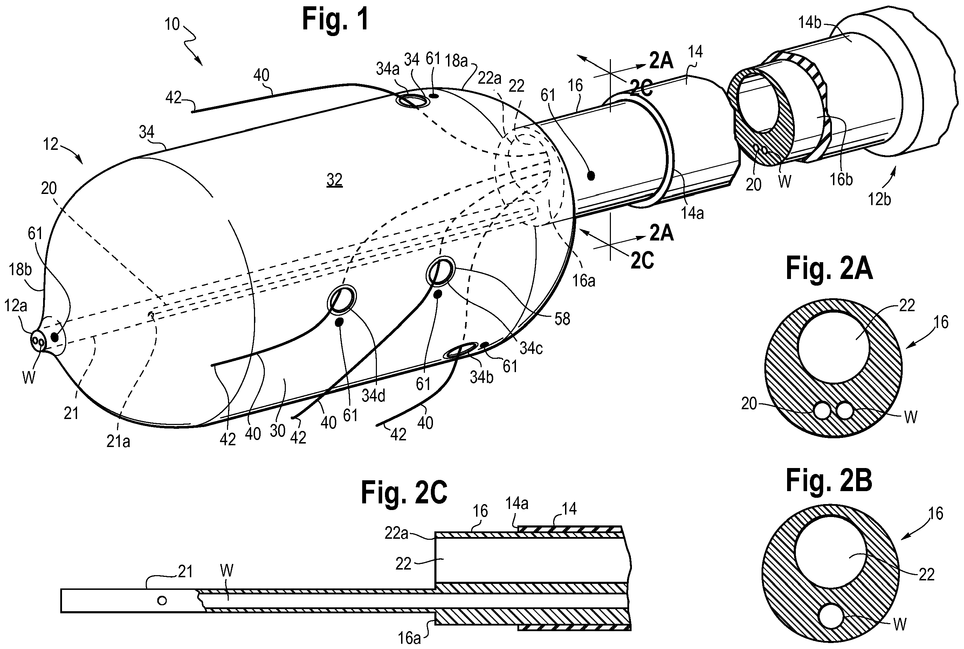

Figures 1-10 show a system 10 including a balloon catheter 12 and a delivery sheath 14 for delivering and deploying the balloon catheter 12 within a patient's vasculature at a desired location. As used herein, references to an insertion end refer to the end of a device or component that is inserted first into the patient and that is opposite an operator end, which is the end that typically remains out of the body.

Figure 1 is a schematic illustration of a balloon 18 in an expanded or partially expanded state that includes holes 34 allowing for additional balloons to pass through at least partially through a wall 30 of the balloon 18. These additional balloons are not shown in Figure 1 in order to illustrate the holes 34 and other structure. Figure 1 therefore illustrates the balloon 18 in an expanded condition, but in use, the balloon 18 typically remains in a compressed position prior to the additional balloons being delivered through the holes 34 to seal the holes 34. However, in some approaches, the balloon 18 may be expanded into a partially expanded state to aid in the introduction of the further balloons, as further described below.

The delivery sheath 14 includes an insertion end 14a and an operator end 14b. The balloon catheter 12 likewise includes an insertion end 12a and an operator end 12b.

The balloon catheter 12 includes a main tubular shaft 16 that extends longitudinally between an insertion end 16a and an operator end 16b, and an inflatable balloon 18 bonded to the shaft 16 near the insertion end 16a of the shaft 16. As shown in Figure 1, the insertion end 12a of the overall balloon catheter 12 is disposed further away from the operator than the insertion end 16a of the shaft 16.

The shaft 16 defines an inflation lumen 20 that is in fluid communication with an interior cavity of the balloon 18. In one approach, the inflation lumen 20 may be defined by the shaft 16 and a tube 21 that extends from the insertion end 16a of the shaft 16 and into the interior of the balloon 18 and to the insertion end 12a of the balloon catheter 12. The shaft 16 and the tube 21 may define an inflation port 21a that delivers inflation fluid from the inflation lumen 20 into the interior of the balloon 18, as shown in Figure 1. In one approach, the tube 21 may be a separate component that attaches to the end of the shaft 16, as illustrated in Figure 1. Alternatively, as shown in Figure 2C, the tube 21 may be an integral extension of the shaft 16. In one approach, as shown in Figure 2C, the tube 21 may provide support for the balloon catheter 12, and may include the guidewire lumen W, but where inflation fluid is provided through another lumen such as a delivery lumen 22, as described below.

The shaft 16 further defines a delivery lumen 22 extending longitudinally through the shaft 16 and configured to allow other system components to be housed therein or delivered therethrough. The catheter 12 and shaft 16 may be delivered with or without the use of a guidewire. In one another approach, the balloon catheter 12 may include a guidewire lumen W formed in the shaft 16 and extending through the tube 21 or other similar support structure that extends through the balloon 18, as shown in Figures 1-3. It will be appreciated that the various lumens can be arranged in a variety of ways in the shaft 16 and the through the balloon 18, such that the delivery lumen 22 opens into the interior of the balloon 18 and the inflation lumen 20 can provide inflation fluid to the interior of the balloon, with the guidewire lumen W extending through the balloon 18 and isolated from the inflation lumen 20. One example of a lumen arrangement is shown in Figure 2A, which shows a cross-section of the shaft 16 and the lumens extending therethrough, including guidewire lumen W, delivery lumen 22 and inflation lumen 20.

Figure 2B illustrates an embodiment where the shaft 16 includes the delivery lumen 22 and a guidewire lumen W, but without a separate inflation lumen. In this embodiment, inflation fluid may be delivered through the delivery lumen 22. The guidewire lumen W extends through the tube 21 in this embodiment, as shown in Figure 2C.

In one embodiment, the delivery lumen 22 is sized to be larger/wider than the inflation lumen 20. In particular, the delivery lumen 22 is sized to be wide enough to facilitate delivery of additional balloons through the delivery lumen 22, as well as multiple guidewires for each of the additional balloons, as further described below.

The balloon 18, attached to the insertion end 12a of the catheter 12 as well as to the insertion end 16a of the shaft 16, is preferably in the form of a compliant balloon, meaning that the balloon 18 will typically take the shape of the vessel in which it is deployed once inflated. The balloon 18 is preferably sized to correspond generally to the size of the vessel to which the balloon 18 will be delivered and inflated. The balloon 18 being in the form of a compliant balloon allows the balloon 18 to be inflated to occlude or fill a target blood vessel while limiting instances where the balloon 18 may cause further damage to the vessel wall when inflated. The compliant balloon 18, when inflated, will tend to take the shape of the blood vessel due to its compliant structure. The compliant, or semi-compliant in another approach, balloon 18 helps the balloon 18 accommodate variation in the vascular anatomy that may vary from patient to patient.

In the case of a traditional inflatable balloon, the balloon wall is typically intact such that the balloon will retain the inflation fluid that is introduced into the cavity defined by the balloon to inflate the balloon.

As shown in Figure 1, the balloon 18 includes the wall 30 that defines an interior cavity 32 therein. The interior cavity 32 is in fluid communication with the inflation lumen 20 of the catheter 12 and the delivery lumen 22 of the shaft 16, such that inflation fluid can be introduced into the interior cavity 32 via the inflation lumen 20 or the delivery lumen 22 to inflate and expand the balloon wall 30 in a manner known in the art, as well as allowing further medical devices to be introduced into the interior cavity 32 via the delivery lumen 22.

Furthermore, the balloon 18 defines one or more holes 34, or punctures or passageways or the like, in the wall 30 that permit the passage of additional structure through the wall 30. Accordingly, with the holes 34 extending through the wall 30, the balloon 18 differs from a traditional balloon in that inflation fluid introduced in a balloon with holes would leak out of the balloon absent other structure that will seal the holes. In the present approach, such structure is provided in the form of additional balloons, which are further described below. In one approach, the balloon wall 30 may include a reinforcing band 58, further described below, that surrounds each of the holes 34 to provide reinforcement to the holes 34 in response to additional balloons being inserted through the holes 34.

In one approach, the holes 34 are generally small. Exemplary holes may be about 2-4 mm in width. The size of the holes 34 are preferably selected to be smaller than the size of the ultimate structure that will be extended through the hole 34, such that after the structure is extended through the hole 34 and left in place, the holes 34 will be generally sealed due to the larger size of the inserted structure exerting a radially outward force on the holes 34, such that the interior cavity 32 within the balloon 18 may still be inflated in response to the introduction of inflation fluid. Accordingly, in one approach, 2-4 mm sized holes are one preferred sizing to accommodate a further balloon that inflates to about 8 mm in width, for example.

In one approach, the balloon 18 may include four holes 34a, 34b, 34c, and 34d. The holes 34a-d are located on the balloon 18 such that their location will typically correspond to the general location of branch vessels in the target delivery and deployment area. For example, the four-hole arrangement may be used in the abdominal aorta near the left and right renal arteries (LRA and RRA) and the supermesenteric artery (SMA) and celiac artery (CA). The SMA and CA are typically disposed above a patient's renal arteries, such that they are between a patient's renal arteries and the heart.

Thus, for a balloon 18 that is designed and arranged to be delivered to this area of the patient, holes 34a and 34b can be arranged on laterally opposite sides of the balloon 18 to accommodate the LRA and RRA, with holes 34c and 34d being disposed longitudinally offset from the holes 34a and 34b and generally on the same lateral side of the balloon 18 as each other. Differing anatomy may result in altering the arrangement of the holes 34 as needed. Further, depending on the desired location for introduction and inflation of the balloon 18, additional holes or fewer holes may be used.

The balloon 18 defines a first end 18a and a second end 18b. The first end 18a is preferably attached to the insertion end 16a of the shaft 16, with the second end 18b being bonded to the insertion end 12a of the balloon catheter 12 at the opposite longitudinal end of the balloon 18 opposite the interface between the insertion end 16a of the shaft 16 and the first end 18a of the balloon 18. The delivery lumen 22 includes an opening 22a at the insertion end 16a of the shaft 16 that is in fluid communication with the interior cavity 32 of the balloon, such that wires or other structure can be passed through the delivery lumen 22 and into the interior cavity 32. The delivery lumen 22 therefore could be used for providing inflation fluid as an alternative to the inflation lumen 20 and tube 21, with the tube 21 being used for support rather than inflation, as described above, and the tube 21 may include the guidewire lumen W but remain fluidly isolated from the cavity 32 of the balloon 18, as shown in Figure 1B.

As shown in Figure 1, the system 10 may further include one or more wires 40 for assisting in the delivery of additional structure to the holes 34. The wires 40 will act as guidewires for the additional structure to allow for the additional structure to be routed to the desired hole 34. The number of wires 40 preferably corresponds to the number of holes 34 in the balloon 18. However, it will be appreciated that the number of wires 40 could differ from the number of holes 34 in some cases. Typically, each hole 34 will have a corresponding wire 40 extending through the hole 34.

Thus, in one approach, the wires 40 extend through the delivery lumen 22 and out of the insertion end 16a of the shaft 16 and into the interior cavity 32 defined by the balloon 18. Each individual wire 40 may further extend through a corresponding hole 34 of the balloon 18 and out of the interior cavity 32 of the balloon 18, such that a terminal end 42 of the wire 40 is disposed outside of the interior cavity 32. The wires 40 are preferably arranged in a pre-loaded state, such that they extend though the holes 34 of the balloon while the balloon 18 is housed within the delivery sheath 14 prior to insertion into the body. The wires 40 may be preloaded as packaged and provided to the doctor in a pre-loaded state, or the wires 40 may be loaded by the doctor prior to delivery of the catheter 12 into the patient. In either case, the wires 40 are preloaded in the catheter 12 in a delivery configuration prior to insertion into the patient. Thus, by being pre-loaded, the wires 40 may already extend out of the holes 34 and will not need to be routed through the generally small holes 34 of the balloon 18 after the balloon 18 is exposed from the sheath 14 and delivered to the desired delivery area. Accordingly, the wires 40 being pre-loaded will result in the wires 40 extending through the holes 34 prior to the balloon 18 being inflated.

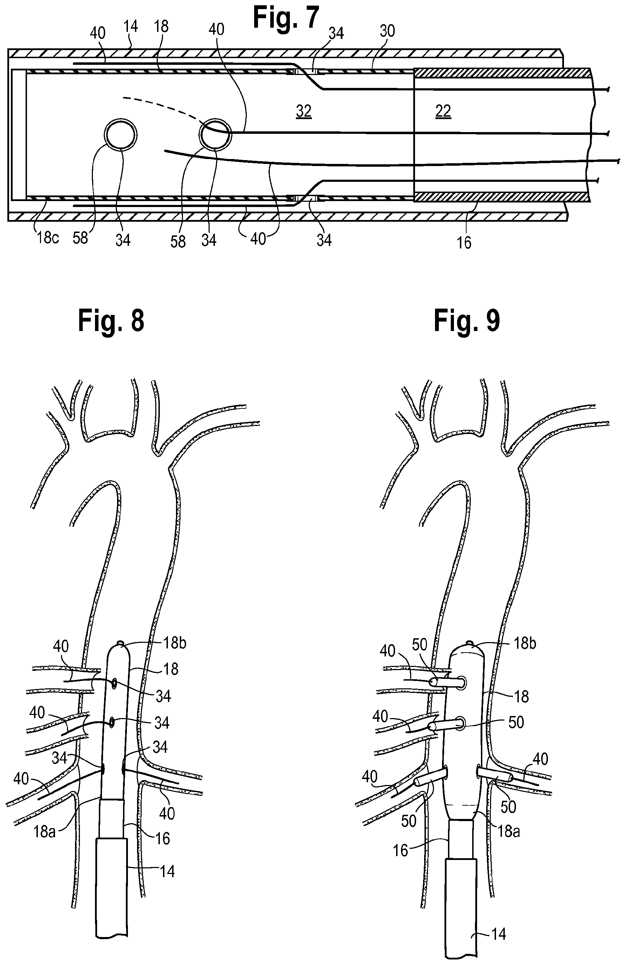

In another approach, the pre-loaded wires 40 may terminate within the balloon cavity 32 when the balloon catheter 12 is delivered in the delivery configuration, and the wires 40 may be carefully routed through the holes 34 after the balloon 18 has been exposed within the body lumen. In this approach, the balloon 18 may be partially inflated to increase the size of the cavity 32 to aid in routing the wires 40. In another approach, the wires 40 may not be pre-loaded and may be introduced through the balloon catheter 12 and into and through the cavity 32 after the balloon 18 has been delivered. Figure 7 illustrates wires 40 both extending through the holes 34 in the preloaded state and terminating within the cavity 32 in the preloaded state.

Thus, with the wires 40 extending from the delivery lumen 22 through the cavity 32 and out of the balloon 18 to the exterior of the balloon 18, additional components can be delivered along the wires 40 and will be routed to the corresponding hole 34 through which the wires 40 extend.

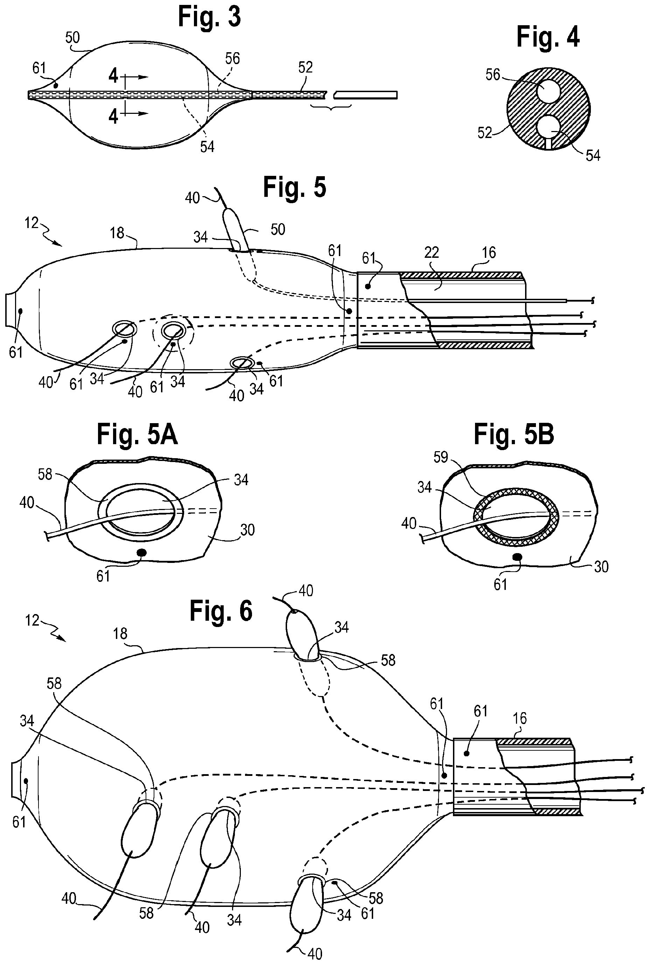

With reference now to Figures 3-6, the system 10 further includes one or more "additional," "secondary," or "daughter" balloons 50 that are configured to be delivered through the delivery lumen 22 over the wires 40 and into engagement with the holes 34 defined by the balloon 18.

Figures 3 and 4 shown examples of daughter balloons. The daughter balloons 50 may be attached to a shaft 52 having an inflation lumen 54 and a wire lumen 56 in the manner of a traditional balloon catheter. The inflation lumen 54 provides inflation fluid to the interior of the balloon 50 to inflate and expand the balloon 50 in a manner known in the art, and the balloon 50 and shaft 52 are deliverable over a wire via the wire lumen 56 in a known manner as well. Figure 4 illustrates one example of a lumen arrangement, showing the shaft 52 in cross-section. However, it will be appreciated that other arrangements of the lumens could be used, such as a dual lumen design for the inflation lumen 54, or a coaxial lumen design where the wire lumen 56 is disposed coaxially within the inflation lumen 54.

Thus, with reference to Figure 5, in one approach, individual daughter balloons 50 are deliverable over individual wires 40 through the delivery lumen 22 of the shaft 16 and into engagement with the hole 34 that corresponds to the wire 40 over which the particular individual balloon 50 was delivered. Figure 5 illustrates a first daughter balloon 50 being delivered to one of the holes 34 over the wire 40 that extends through the hole 34. Figure 5 further illustrates the other holes 34 having wires 40 extending therethrough. Figure 5 illustrates the balloon 18 in a partially inflated state, however delivery of the daughter balloons 50 may occur when the balloon 18 is still in a compressed state and prior to any inflation. It may be difficult to inflate the balloon 18 effectively prior to delivery of each of the daughter balloons 50 due to the holes 34 being present in the balloon 18.

The daughter balloons 50 may be made from traditional medical balloon materials, and can be compliant or semi-compliant, depending on the needs of the user. The size of the daughter balloons 50 can also be selected to correspond to a patient's particular anatomy. As described above with respect to the holes 34, the expanded width of the balloon 50 is preferably greater than the size of the hole 34, such that when the balloon 50 is expanded into engagement with the hole 34, the edge of the hole 34 will seal against the outer wall of the daughter balloon 50. The compliant nature of the balloon 18 will allow the hole 34 to stretch to accommodate the expanded outer width of the daughter balloon 18. In one approach, the daughter balloons 50 are more rigid than the balloon 18, such that the daughter balloons 50 will stretch the holes 34 in the balloon 18, as shown in Figure 5. In another approach, the daughter balloons 50 may be less rigid, and in this case the holes 34 may not stretch, and instead the daughter balloons 50 would expand further on each side of the hole 34 than in the hole 34, such that the diameter of the daughter balloon 50 on either side of the hole 34 is greater than the diameter of the hole 34, thus taking on a somewhat hourglass shape, as shown in Figure 6.

The balloons 50 are preferably delivered over the wires 40 in a sequential manner, such that the delivery lumen 22 of the shaft 16 can be sized to accommodate the number of preloaded wires 40 in addition to allowing a single balloon 50 to be delivered through the lumen 22, which keeps the overall width of the shaft 16 small. However, it would also be possible to increase the size of the delivery lumen 22 to allow for delivery of more than one balloon 50 at a time side-by-side, but this would also increase the width of the shaft 16 to a size larger than one where balloons 50 are delivered sequentially.

Figure 6 illustrates the balloon 18 in a fully inflated state after each of the daughter balloons 50 have been delivered to the corresponding holes 34 over the corresponding wires 40. The balloons 50 have been inflated and have created a seal with the holes 34, thereby sealing off the cavity 32 inside the balloon 18 such that the balloon 18 may be inflated. Figure 6 illustrates the example of the daughter balloons 50 taking on an hourglass shape after inflation, as described above.

Due to the expansion of the balloons 50 while extending through the holes 34 to create the seal, the balloon 34 may also include the reinforcing band 58 disposed around the edge of the holes 34, as shown in Figure 5A. The reinforcing band 58 can help prevent tearing of the balloon 18 at the location of the holes 34 when the holes 34 are stretched, and can also help provide a seal against the expanded daughter balloon 50. As shown in Figure 5A, the reinforcing band 58 can be in the form of an additional layer of balloon material that is bonded or adhered to the area surrounding the hole, or it could be in the form of an applied coating or curing adhesive. In another approach, as shown in Figure 5B, a mesh material 59 may be embedded in the wall 30 of the balloon 18 around the holes 34.

When the daughter balloons 50 are expanded into a sealing engagement with the holes 34 of the balloon 18, the balloon 18 will be generally sealed from inflation fluid leaking out of the balloon 18 when the balloon 18 is inflated. It has been found that the use of 8x20 mm sized daughter balloons 50 inserted into 2-4 mm holes 34 in the balloon 18 allows the balloon 18 to hold greater than 1 atm of pressure when inflation fluid is introduced into the cavity 32 to inflate the balloon 32. As shown in Figure 5, the balloon 18 and the holes 34 thereof, the daughter balloons 50, and shaft 16, as well as other structure described above, can each include radiopaque markers 61 disposed at various selected locations to aid in locating the balloon 18 and the various corresponding and cooperating structure at the desired location within the patient's anatomy. For example, markers 61 may be located at each of the holes 34 to assist in positioning the balloon 18 so that the wires 40 and daughter balloons 50 may be routed to the desired branch vessels. Similarly, the wires 40 may be made of a radiopaque material.

The daughter balloons 50 can be used to cannulate various branch vessels adjacent the delivered location of the main balloon 18. This can be performed quickly and easily due to the pre-loaded wires 40 that extend through the balloon 18 in its delivery state. The wires 40 are moveable relative to the shaft 16 and the holes 34 of the balloon 18, such that after the balloon 18 and wires 40 have been delivered, the wires 40 can be individually extended into the desired branch vessel prior to delivering the daughter balloon 50. Accordingly, the daughter balloon 50 will enter the desired branch vessel along the wire 40.

The system 10 has a delivery state and a deployed state. With reference to Figure 7, in the delivery state, the balloon 18 and shaft 16 and wires 40 are disposed within the delivery sheath 14 and covered by the delivery sheath 14. The wires 40 are pre-loaded in the balloon 18. Figure 7 shows three wires 40 extending through the holes 34 in the balloon 34 such that they extend through the holes 34 and to the exterior of the balloon 18 while in the delivery state. Figure 7 also illustrates one wire 40 terminating within the balloon cavity 32. It will be appreciated that all of the wires 40 may extend out of the holes 34 in the delivery state, all of the wires 40 may terminate with the cavity 32 in the delivery state, or some of the wires 40 may extend out of the holes 34 and others of the wires 40 may terminate within the balloon cavity 32.

In the deployed state, shown in Figure 8, the delivery sheath 14 is retracted relative to the balloon 18, shaft 16, and wires 40, thereby exposing the balloon 18 and the wires 14 to the surrounding vasculature. From this deployed state, the wires 40 may be routed into the desired branch vessels, and the balloons 50 may be introduced as described above and the balloon 18 may be inflated.

As shown in Figure 8, upon the balloon 18 being exposed by retracting the sheath 14 at the desired location, the wires 40 are routed into the desired adjacent branch vessels. As shown in Figure 9, following positioning of the wires 40, the daughter balloons 50 are delivered over the wires 40 and into the branch vessels while also being disposed within the holes 34 of the balloon 18. The balloons 50 are preferably delivered sequentially. However, as described above, in another approach multiple balloons 50 may be delivered side-by-side or otherwise together if the delivery lumen 22 is wide enough to accommodate multiple daughter balloons 50 in that arrangement.

After delivering the daughter balloons 50 over the wires 40 and into the holes 34 of the balloon 18, the daughter balloons 50 are inflated. The daughter balloons 50 can be inflated sequentially after delivering each individual balloon 50, or multiple balloons 50 may be inflated at the same time after delivering multiple balloons 50. In another approach, the balloons 50 could each be inflated at the same time after some or all of the balloons 50 have been delivered.

As the balloons 50 are inflated, they can be inflated to provide support to the branch vessels. In another approach, the balloons 50 may be positioned at different areas outside of the balloon 18 outside of a branch vessel. For example, one or more of the balloons 50 could be positioned against the main vessel wall.

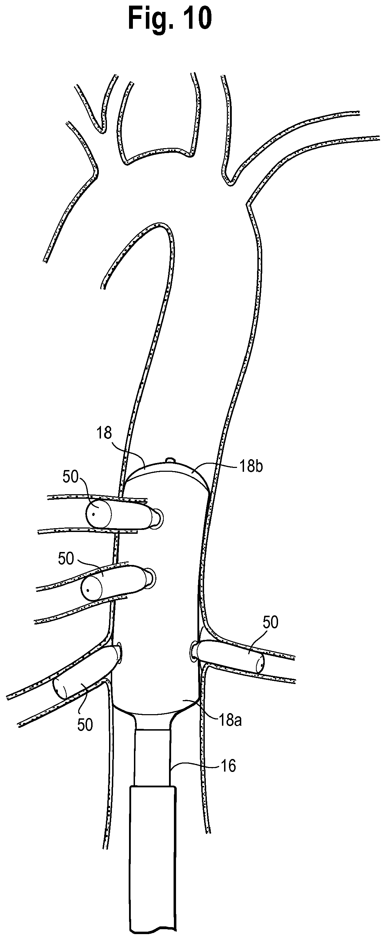

As shown in Figure 10, after each of the daughter balloons 50 have been inflated, the main balloon 18 is inflated and expanded into engagement with the main vessel wall. The previous inflation of the daughter balloons 50 provides a sealing and filling of the holes 34, such that the main balloon 18 will sufficiently inflate.

In one approach, the balloon 18 is compliant, and the daughter balloons 50 are minimally compliant, meaning that the daughter balloons 50 can inflate to a predefined shape, causing the balloon 18 to stretch in response to inflation of the daughter balloons 50. Therefore, when the balloon 18 is inflated, it will tend to conform to the shape of the vessel wall as well as around the daughter balloons 50. In another approach, the daughter balloons 18 may be compliant and will take the shape of the vessel in which they are deployed, and inflation of the balloon 18 may alter the shape of the daughter balloons 50, depending on whether the balloon 18 or daughter balloons 50 are more rigid relative to each other.

The main balloon 18 can be cycled between an inflated and deflated position to open up a blocked blood vessel. In this case, the daughter balloons 50 provide support to the branch vessels as the main balloon 18 is inflated and deflated. While the balloon 18 has been described as being compliant and conforming to the shape of the vessel, it will be appreciated that the balloon 18 may still be arranged to have sufficient rigidity when inflated to open up a blocked vessel.

In the case of aortic dissection, such as type B dissection, the daughter balloons 50 in their inflated state provide support to the branch vessels, while the inflation of the main balloon 18 provides support within the true lumen, thereby allowing for filling the false lumen with embolic material.

It will be appreciated that the above arrangement may be used in various other situations where balloon support for a main vessel and branch vessels is desired.

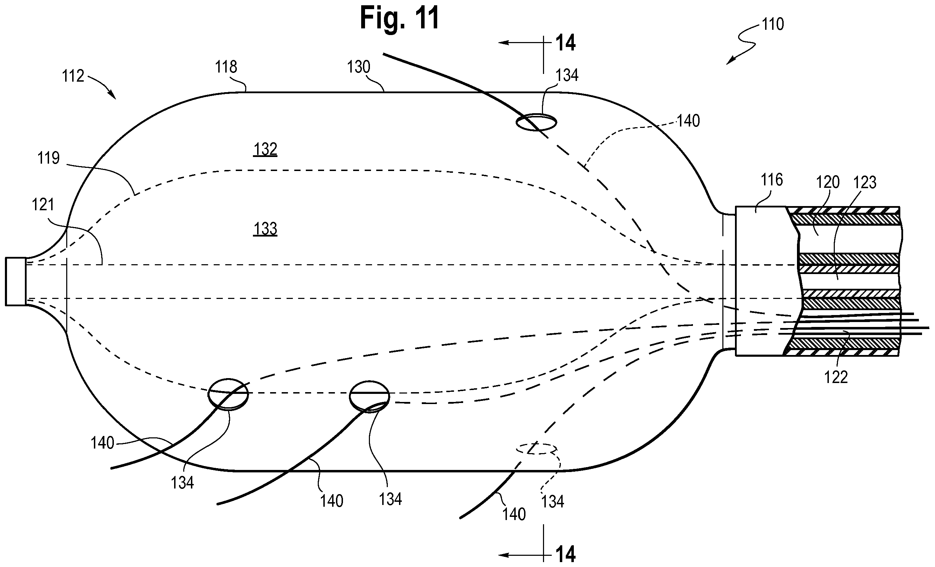

With reference to Figures 11-20, in another embodiment, a system 110 can include a balloon catheter 112 having a first compliant balloon 118 and a second compliant balloon 119, where the first compliant balloon 118 completely subsumes the second compliant balloon 119. Put another way, the first balloon 118 is an outer balloon, and the second balloon 119 is an inner balloon. The balloon 118 has a similar structure and functionality to the balloon 18 described above, and the descriptions of the balloon 18 can be equally applied to the balloon 118.

As shown in Figure 11, the first balloon 118 and the second balloon 119 define a space or cavity 132 between them. The first balloon 118 includes holes 134 in an outer wall 130 that allow fluid communication between the cavity 132 and the area outside the balloon 118.

A shaft 116 includes an inflation lumen 120 in fluid communication with the cavity 132. The shaft 116 further includes a second inflation lumen 123 that is in fluid communication with a cavity 133 defined within the second balloon 119.

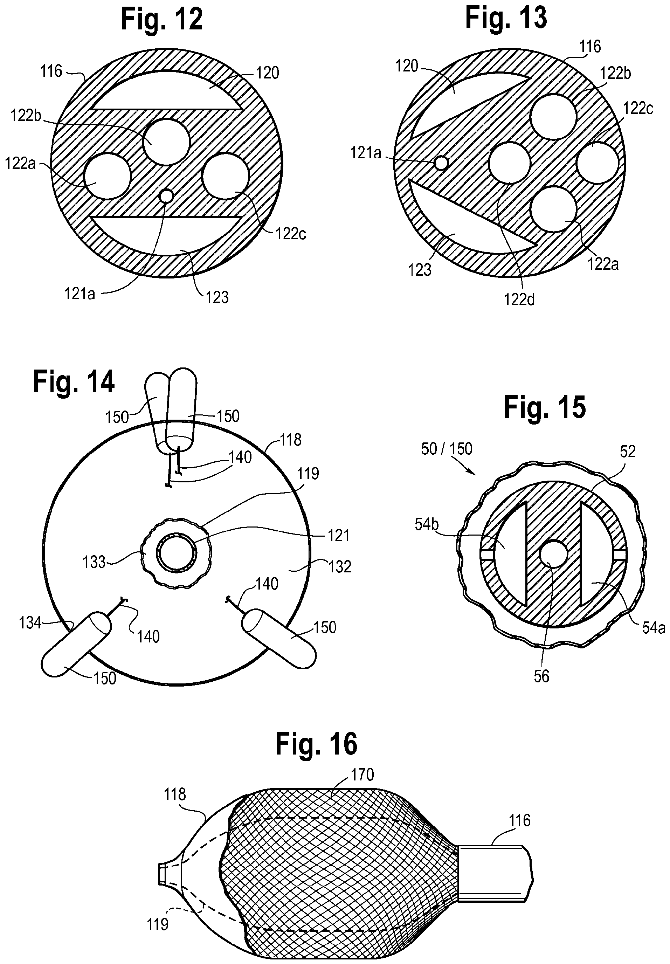

The catheter 112 may further include a tubular support 121 that extends through the cavity 133 of the inner balloon 119. The catheter 112 may include a wire lumen 121a (shown in Figures 12 and 13) extending therethrough, which extends fully through the catheter 112 such that the catheter 112 can be delivered over a guidewire via the wire lumen 121a.

The shaft 116 includes a delivery lumen 122, similar to the lumen 22 described above. In another approach, the delivery lumen 122 could be in the form of multiple daughter balloon delivery lumens 122a, 122b, 122c, 122d for separating individual wires 140 used for delivering daughter balloons 150, as well as the wire lumen 121 a for delivering the shaft 116 and catheter 112 to a desired location. Examples of the multi-lumen arrangement can be seen Figures 12 and 13.

Figure 14 illustrates the inner balloon 119 and the outer balloon 118, with daughter balloons extending through the outer balloon 118, and the inner balloon 119 in a deflated state, and further illustrating the wire lumen 121a extending through the support 121.

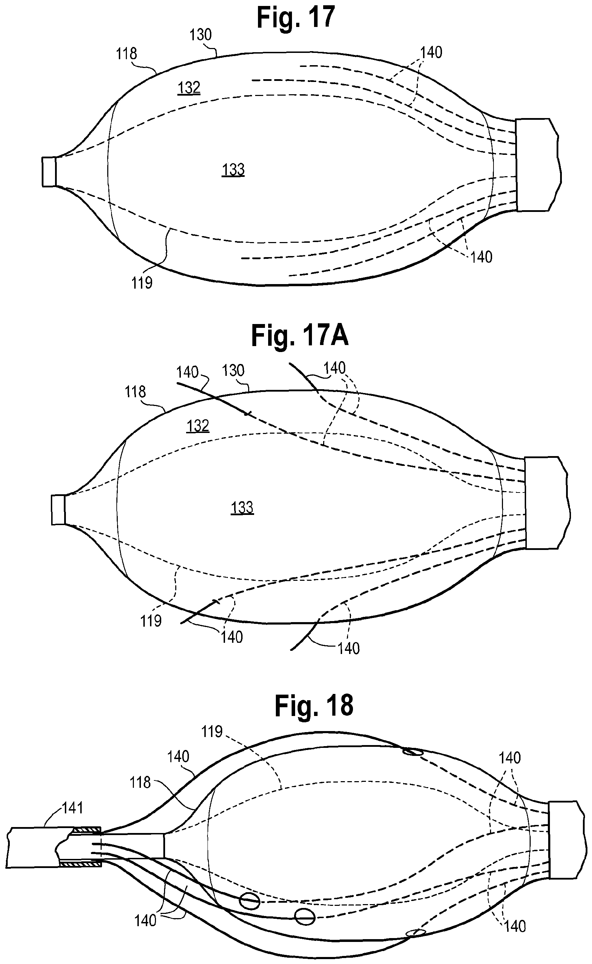

The wires 140 can be pre-loaded similar to the wires 40. However, in another approach, as shown in Figure 17, the balloon 118 can be configured without any pre-formed holes through the wall 130. In this approach, the wires 140 can be preloaded such that their ends terminate within the cavity 132 but do not extend outside the cavity 132. The wires 140 themselves can be used to puncture the wall 130 of the balloon 118, as shown in Figure 17A, to thereby extend the wires 140 out of the balloon 118.

Thus, the balloon 118 and the wires 140 can be used to accommodate patient anatomy that may differ from a traditional anatomy, or to allow for delivery of the balloons 118, 119 and the daughter balloons 150 to an atypical location. This configuration can also be used for traditional or expected anatomy arrangements, but when it may be unclear whether a branch vessel would benefit from receiving a daughter balloon. Accordingly, this configuration provides added flexibility in determining the quantity and location of the holes 134a that are defined in the wall 130 of the balloon 118 after the balloon 118 has been delivered.

With reference to Figure 18, in the case where the wires 140 are preloaded and extend through pre-formed holes 134, the terminal ends of the wires 140 may be detachably fixed to the balloon catheter 112 outside of the balloon 118. The wires 140 can be extended into a sleeve 141 for retention, and can be pulled back and separated for subsequent routing to a desired location within the body vessel.

In this approach, the terminal ends extending outside of the balloon 118 can be retained such that they are not loose during delivery. To detach the wires 140, the wires 140 can be pulled back toward the user and then fed into the desired branch vessel or other desired location. This securement arrangement could also apply to the wires 40 described above.

To deliver the daughter balloons 150, the process is similar to that described above regarding the daughter balloons 50. However, for the system 110, the balloon 118 is partially inflated prior to delivery of the daughter balloons 150 to separate the balloon 118 from the balloon 119, thereby opening up the cavity. This partial inflation helps the balloons 150 to move through the cavity 132 between the balloons 118 and 119. Moreover, this partial (or further) inflation is desired in the case where the balloon 118 does not include holes and the wires 140 are used to puncture the balloon 118 on demand. In these instances, it is desirable that care be taken to avoid puncturing the inner balloon 119.

The inner balloon 119 is the desired balloon for providing the main inflation for the system 110, while the outer balloon 118 provides the structural support for the daughter balloons 150. Thus, once the daughter balloons 150 have been delivered to the desired location and inflated, the inner balloon 119 is inflated to fill the blood vessel. The compliant nature of both the outer balloon 118 and the inner balloon 119 causes both balloons 118 and 119 to take the shape of the vessel wall and support the vessel wall.

However, the outer balloon 118 could also be used to provide the main inflation in the event of a problem with the inner balloon 119. As described above with respect to the balloon 18, the expansion of the daughter balloons 150 into the holes 134 will seal the holes 134 to a degree sufficient for inflation. However, a balloon lacking any holes or with minimal sealing interfaces may provide additional inflation pressure and may desirable in some applications. The "balloon-in-a-balloon" arrangement described with respect to the balloons 118 and 119 provides that added functionality relative to the single balloon arrangement described above with respect to the balloon 18.

In addition to using the daughter balloons 150 (or 50) to fill branch vessels, the daughter balloons 150 can be used to push the main balloon 118 (or 18) away from the vessel wall on one side, thereby providing a flow path along one side while the opposite side remains pushed against the vessel wall. To provide this sort of pushing ability, the daughter balloons 50 or 150 are preferably made from a minimally compliant material, such that they will be inflated to a predetermined shape and will be less apt to conform to the shape of the surrounding anatomy.

The daughter balloons 50 and 150 have been described as having an inflation lumen 54 and a wire lumen 56. As shown in Figure 15, in one approach, the inflation lumen 54 is in the form of a dual lumen 54a and 54b, such that inflation can occur even in a situation where the shaft of the daughter balloon 50 or 150 is kinked.

As shown in Figure 16, in one approach, the outer balloon 118 can have an additional material applied to the outside surface for reinforcement, such as an interwoven mesh 170 or the like. This mesh 170 defines a plurality of cells, and can be coated with silicone, thoralon, or the like. Thus, puncture of the balloon 118 by the wires 140 to define the holes 134a can be isolated and potential tearing can be limited to the connection of the balloon 118 to the mesh 170.

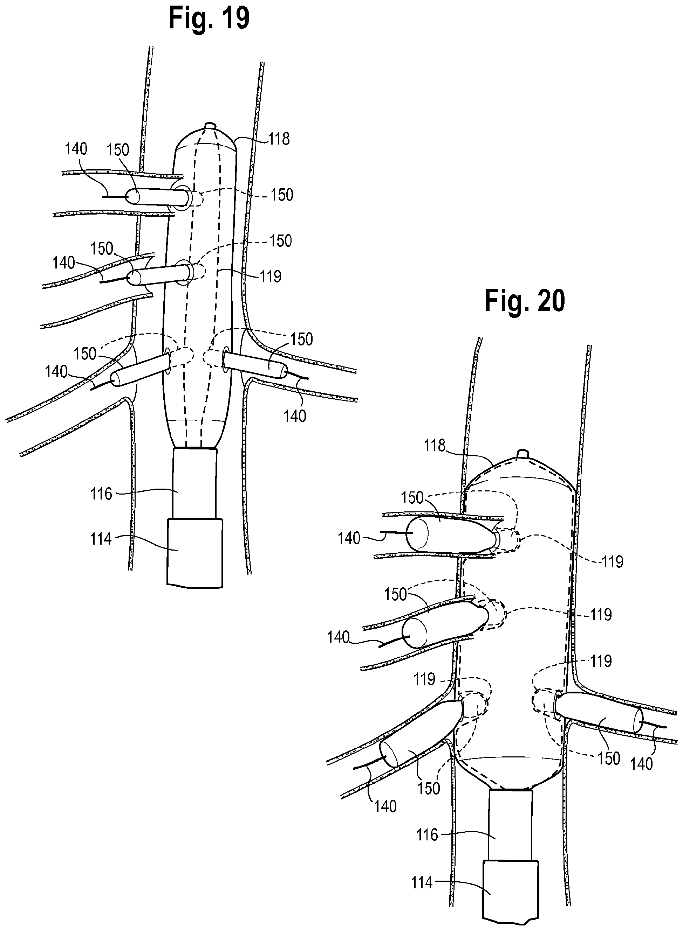

With reference to Figures 19 and 20, the system 210 can be delivered to the desired body vessel in a manner similar to that described above with relation to the system 10.

The balloons 118 and 119 are delivered to the desired location where lumen support or dilation is desired. The wires 140, being already extended through the holes 134 or disposed within the cavity of the balloon 118, are routed through the holes 134 and into the desired location, such as within a branch vessel. The daughter balloons 150 are delivered over the wires 140 and into the corresponding branch vessel, as well as extending through the holes 134. The daughter balloons 150 are inflated into engagement with the holes 134 such that the daughter balloons 150 are retained by the outer balloon 118 via the holes 134. The daughter balloons 150 may seal against the holes. However, in this embodiment, the daughter balloons 150 may not fully seal against the holes 134. In the event of the lack of a full seal, inflation may still be provided by the inner balloon 119.

With the daughter balloons 150 retained by the outer balloon 118, as shown in Figure 19, the inner balloon 119 is then inflated and expanded into engagement with the body vessel wall, as shown in Figure 20. The inner balloon 119 does not include any holes, so inflation of the inner balloon 119 is not dependent on sufficient sealing between the daughter balloons 150 and their engagement with the holes 134 of the outer balloon 118.

With the balloons 118, 119 being compliant balloons, the balloons 118 and 119 will generally conform to the shape of the body vessel, as well as to the shape of the minimally compliant daughter balloons 150.

As described above, the delivery lumen 122 can be in the form of multiple dedicated delivery lumens 122a, b, c, and d. Each of these dedicated lumens can include a corresponding wire 140 that extends into the cavity 132 and optionally through a pre-defined hole 134. The daughter balloons 150 are deliverable through each of these dedicated lumens 122, allowing for delivery of multiple daughter balloons 150 at the same time. However, it will be appreciated that the balloons 150 could also be delivered sequentially through a single delivery lumen.

If additional inflation is desired beyond the inflation provided by the inner balloon 119, the outer balloon 118 may be inflated as well, depending on the sufficiency of the seal between the daughter balloons 150 and the holes 134 in the outer balloon 118. Thus, the inner balloon 119 provides the bulk of the inflation pressure, with the outer balloon 118 providing additional inflation if desired.

The balloons 118, 119 may be repeatedly inflated and deflated, while the daughter balloons 150 can remain inflated, due to the dedicated inflation lumens for each of the daughter balloons 150.

Throughout this specification various indications have been given as to preferred and alternative examples and aspects of the invention. However, the foregoing detailed description is to be regarded as illustrative rather than limiting and the invention is not limited to any one of the provided aspects. It should be understood that it is the appended claims, including all equivalents, that are intended to define the spirit and scope of this invention.

The disclosures in United States patent application number US-15/631,772, from which this application claims priority, and in the abstract accompanying this application are incorporated herein by reference.

## Claims

1. A medical system comprising:
a balloon catheter including a shaft and an inner balloon bonded to the shaft and an outer balloon bonded to the shaft and subsuming the first balloon;
a first inflation lumen extending through the shaft and in fluid communication with an interior cavity of the inner balloon;
an intermediate space between an inner surface of the outer balloon and an outer surface of the inner balloon;
at least one delivery lumen extending longitudinally through the shaft and in fluid communication with the intermediate space, the delivery lumen configured to deliver medical devices through the shaft and into the intermediate space;
at least one hole in an outer wall of the outer balloon; and
at least one daughter balloon moveable through the at least one delivery lumen and configured to engage the at least one hole of the outer balloon when the daughter balloon is inflated and expanded.

2. A system according to claim 1, comprising a second inflation lumen extending within the shaft and in fluid communication with the intermediate space, wherein the inner balloon and outer balloon are independently inflatable via the first inflation lumen and the second inflation lumen, respectively..

3. A system according to claim 1 or 2, comprising at least one wire extending through the at least one delivery lumen into the intermediate space and out of intermediate space through the at least one hole, and the daughter balloon is deliverable over the at least one wire into engagement with the outer balloon.

4. A system according to claim 3, wherein a terminal end of the at least one wire is detachably retained on the shaft outside of the outer balloon.

5. A system according to any preceding claim, wherein the at least one hole is pre-defined in the outer balloon.

6. A system according to any preceding claim, wherein the inner balloon is a compliant balloon.

7. A system according to any preceding claim, wherein the at least one hole is defined by the at least one wire extending through the outer balloon.

8. A system according to any preceding claim, wherein the at least one delivery lumen comprises multiple delivery lumens.

9. A system according to claim 8, wherein the at least one daughter balloon comprises multiple daughter balloons, and individual ones of the daughter balloons are deliverable via a corresponding one of the multiple delivery lumens.

10. A system according to any preceding claim, wherein inflation of the inner balloon expands the outer balloon.

11. A system according to any preceding claim, wherein inflation of the daughter balloon within the hole of the outer balloon seals the hole of the outer balloon.

12. A medical system comprising:
a balloon catheter including a shaft and an inner balloon bonded to the shaft and an outer balloon bonded to the shaft and subsuming the first balloon;
a first inflation lumen extending through the shaft and in fluid communication with an interior cavity of the inner balloon;
an intermediate space between an inner surface of the outer balloon and an outer surface of the inner balloon;
at least one delivery lumen extending longitudinally through the shaft and in fluid communication with the intermediate space, the delivery lumen configured to deliver medical devices through the shaft and into the intermediate space;
at least one daughter balloon moveable through the at least one delivery lumen; and
at least one wire extending through the at least one delivery lumen and into the intermediate space;
wherein the at least one daughter balloon is deliverable into the intermediate space over the at least one wire;
wherein the system includes a delivery configuration and a deployed configuration, where the inner balloon and outer balloon are in a compressed state in the delivery configuration; and
wherein, in the deployed configuration, at least one of the inner balloon or the outer balloon are inflated, and the at least one daughter balloon extends through a corresponding hole in the outer balloon and is supported by the outer balloon when the daughter balloon is inflated.

13. A system according to claim 12, wherein the hole corresponding to the at least one daughter balloon is pre-defined through the outer balloon in the delivery configu ration.

14. A system according to claim 12 or 13, wherein the at least one wire terminates within the intermediate space in the delivery configuration, and the hole corresponding to the at least one daughter balloon is defined in response to piercing the outer balloon with the at least one wire in the deployed configuration.

15. A system according to any preceding claim, wherein the outer balloon includes a reinforcing structure in addition to a compliant material of the balloon.
